(19) Europäisches Patentamt
European Patent Office
Office européen des brevets

(11) Publication number: **0 507 023 A1**

# (12) EUROPEAN PATENT APPLICATION

(21) Application number: **91303039.1**

(51) Int. Cl.$^5$: **C07D 307/33**

(22) Date of filing: **05.04.91**

(43) Date of publication of application:
**07.10.92 Bulletin 92/41**

(84) Designated Contracting States:
**DE FR GB NL**

(71) Applicant: **Tonen Corporation**
**1-1,Hitotsubashi, 1-Chome Chiyoda-Ku**
**Tokyo(JP)**

(72) Inventor: **Suzuki, Sadakatsu**
**1-4-215, Nishitsurugaoka, Ooi-machi**
**Iruma-gun, Saitama-ken(JP)**
Inventor: **Fujii, Takeaki**
**1-4-6, Nishitsurugaoka, Ooi-machi**
**Iruma-gun, Saitama-ken(JP)**
Inventor: **Ueno, Hiroshi**
**398-1, Haneo, Namekawa-machi**
**Hiki-gun, Saitama-ken(JP)**

(74) Representative: **Bawden, Peter Charles et al**
**EXXON CHEMICAL LIMITED EXXON**
**CHEMICAL TECHNOLOGY CENTRE PO Box 1**
**Abingdon Oxfordshire OX13 6BB(GB)**

## (54) Process for producing gamma-butyrolactone.

(57) Gamma-butyrolactone is prepared by the dehydrogenation in the gas phase of 1,4-butanediol in the presence of a catalyst comprising copper, chromium, manganese and barium and in the presence of hydrogen.

EP 0 507 023 A1

The present invention relates to a process for producing gamma-butyrolactone, and more particularly, to a process for producing gamma-butyrolactone by catalytic dehydrogenation of 1,4-butanediol in gas phase in the presence of a catalyst.

Gamma-butyrolactone is a useful compound as a solvent such as for dyes and fiber spinning and also as an intermediate raw material for N-methylpyrrolidone which is a polymerization solvent for polyphenylene sulfide. Therefore, there has been a strong desire for the development of a process for producing gamma-butyrolactone economically and efficiently.

Incidentally, the processes known so far for the production of gamma-butyrolactone include (a) one which involves the oxidative dehydrogenation of 1,4-butanediol in the presence of a palladium, platinum, or silver catalyst, (b) one which involves the catalytic hydrogenation of maleic anhydride or an ester thereof in the presence of a catalyst, and (c) one which involves the dehydrogenation of 1,4-butanediol in the presence of a copper-chromium-manganese catalyst or a copper-chromium-zinc catalyst as disclosed in Japanese Patent Laid-open No. 246173/1986.

However, process (a) has a disadvantage that the catalyst activity is low and the selectivity of gamma-butyrolactone is low; process (b) has a disadvantage that the catalyst life is short in both liquid-phase reaction and gas-phase reaction; and process (c) has a disadvantage that the yield is not necessarily satisfactory.

The present invention is intended to eliminate the low yield and selectivity of gamma-butyrolactone which are the disadvantages of prior art technologies encountered in the production of gamma-butyrolactone from 1,4-butanediol. Accordingly, it is an object of the present invention to provide a process for producing gamma-butyrolactone economically in high yields.

The present inventors carried out a series of researches on the process for producing gamma-butyrolactone from 1,4-butanediol by catalytic dehydrogenation.

As the result, it was found that the yield and selectivity of gamma-butyrolactone remarkably increase if the reaction is performed in gas phase at normal pressure or a light pressure (several $kg/cm^2G$) under a hydrogen stream in the presence of catalyst containing copper, chromium, manganese, and barium. G is gauge pressure. This finding led to the present invention.

Accordingly, the present invention relates to a process for producing gamma-butyrolactone by catalytic dehydrogenation of 1,4-butanediol, which comprises performing the reaction in gas phase in the presence of a catalyst containing copper, chromium, manganese, and barium.

The present invention employs a catalyst which is a reduction product of a mixture of copper oxide-chromium oxidemanganese oxide-barium oxide catalyst. This oxide catalyst is prepared by, for example, a process which consists of dissolving a copper compound (such as copper nitrate) and a chromium compound (such as chromium nitrate) in water, adding for neutralization an aqueous solution of sodium carbonate dropwise with heating and stirring, filtering out solids, incorporating the solids with an aqueous solution of a manganese compound (such as manganese chloride) and an aqueous solution of a barium compound (such as barium chloride), drying and calcining the mixture, and forming the calcined product into a desired shape using a molding machine.

The catalyst used in the present invention has a copper/chromium atomic ratio of 0.4 to 1.8, preferably 0.8 to 1.4.

In addition, the catalyst contains manganese (as metal) in an amount of 1 to 10 parts by weight, preferably 2 to 7 parts by weight, for 100 parts by weight of the total amount of copper and chromium (as metal). Also, the catalyst contains barium (as metal) in an amount of 2 to 20 parts by weight, preferably 2 to 10 parts by weight, for 100 parts by weight of the total amount of copper and chromium (as metal).

The reduction to prepare the catalyst used in the present invention is accomplished by, for example, passing nitrogen containing 2 vol% hydrogen through the oxide catalyst at a gas hourly space velocity (G.H.S.V.) of about 4000 $h^{-1}$ under a pressure of several $kg/cm^2G$ at 140 °C until the catalyst bed gives out no heat any longer, and continuing passing hydrogen for several hours, with the catalyst bed kept at 200 °C after the hydrogen concentration gradually increased to 100 vol%. (G.H.S.V. is expressed in terms of values at normal temperature and normal pressure hereinafter.)

Contact of the mixed gas of 1,4-butanediol and hydrogen with the catalyst may be accomplished by any process properly selected from known ones. For example, it is possible to use a process resorting to the fixed bed system for contact, a process resorting to the moving bed system for contact, and a process resorting to the fluidized bed system for contact. There may be an instance where it is possible to employ batchwise operations for contact.

The reaction involved in the present invention is an equilibrium reaction represented by the equation below.

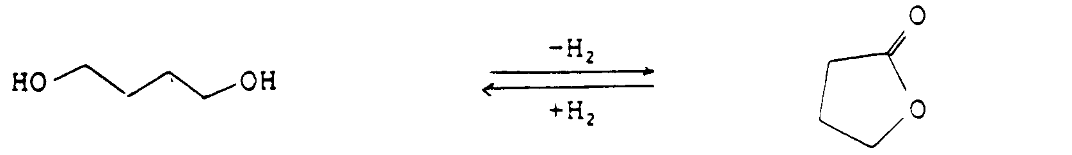


1,4-butanediol gamma-butyrolactone

The above-mentioned equilibrium favors the formation of gamma-butyrolactone when the reaction temperature is high, the reaction pressure is low, and the ratio of hydrogen to 1,4-butanediol is low. However, reactions at an excessively high temperature not only pose problems with coking and copper metal particles sintering, which lead to a reduced catalyst life, but also bring about side reactions, which lower the selectivity of gamma-butyrolactone. That a low reaction pressure is favorable to the formation of gamma-butyrolactone is not always the case. Reactions under pressure give rise to gamma-butyrolactone at a higher rate and hence in a higher yield, so long as the equilibrium is in the gamma-butyrolactone side. Consequently, the reaction in the present invention is preferably carried out under a pressure of several $kg/cm^2G$. In addition, that a low hydrogen/1,4-butanediol ratio is favorable to the formation of gamma-butyrolactone is not always the case. The absence of hydrogen in the system reduces the catalyst life and the presence of hydrogen (as a diluent) helps keep the system in gas phase. Therefore, the reaction in the present invention is preferably performed with an appropriate hydrogen/1,4-butanediol ratio.

For reasons mentioned above, the present invention is preferably put into practice with the reaction temperature at 150-270 °C, more preferably 190-240 °C, with the reaction pressure at 0-8 $kg/cm^2G$, more preferably 0.5-4 $kg/cm^2G$, with the weight hourly space velocity (W.H.S.V.) of 1,4-butanediol at 0.2-16 $h^{-1}$, more preferably 0.4-6.0 $h^{-1}$, and with the hydrogen/1,4-butanediol molar ratio of 0.5-10, more preferably 2-6, which is high enough to keep the system in gas phase.

According to the process of the present invention, it is possible to produce gamma-butyrolactone invariably in a high space time yield and a high selectivity.

The invention will be described with reference to the following examples, which are not intended to restrict the scope of the invention. In the examples, "%" means "% by weight" unless otherwise indicated.

## Example 1

A commercial copper-chromium oxide catalyst ("C-700" made by Sakai Kagaku Kogyo Co., Ltd.) in pellet form (about 1/8 inch in diameter and about 1/16 inch high), which contains 45% CuO, 43% $Cr_2O_3$, 4% MnO, and 2% BaO, was filled as much as 5 cc into a fixed bed reactor (15 mm in diameter and 600 mm long). The catalyst was heated to 140 °C in a nitrogen stream pressurized at 5 $kg/cm^2G$. Then the catalyst was exposed to a nitrogen stream containing 2 vol% hydrogen at 5 $kg/cm^2G$, 140 °C, and a G.H.S.V. of 4000 $h^{-1}$ until there was no heat generation. The temperature was gradually raised to 170 °C. After confirmation that there was no heat generation any longer, the hydrogen content in the nitrogen stream was gradually increased to 100 vol%. After reconfirmation that there was no heat generation any longer, the temperature was gradually raised to 200 °C. The hydrogen stream was kept at 5 $kg/cm^2G$, 200 °C, and a G.H.S.V. of 4000 $h^{-1}$ for 1 hour. In this way, the catalyst was reduced.

With the fixed bed reactor heated at 230 °C, a mixture of 1,4-butanediol (1 mol) and hydrogen (4 mol) was fed under a pressure of 3 $kg/cm^2G$ and at a W.H.S.V. of 3.6 $h^{-1}$ for 1,4-butanediol (or at a total G.H.S.V. of 6790 $h^{-1}$). The reaction product was analyzed by gas chromatography and identified by GC-MS (gas chromatography-mass spectrometry).

It was found that the reaction product contained 97.2 mol% gamma-butyrolactone, with the conversion of 1,4-butanediol being 98.1 mol%, and small amounts of tetrahydrofuran, n-butanol, and 4-hydroxybutylaldehyde. Incidentally, the selectivity of gamma-butyrolactone based on 1,4-butanediol was 99.1 mol%.

## Examples 2 to 6

The same procedure as in Example 1 was repeated for catalyst reduction and reaction, except that the reaction conditions were changed as shown in Table 1. The conversion of 1,4-butanediol, the selectivity of gamma-butyrolactone, and the yield of gamma-butyrolactone are shown in Table 1.

## Comparative Example 1

A commercial copper-chromium oxide catalyst ("G-13" made by Nissan Gardlar Shokubai Co., Ltd.) crushed into granules about 1/8 inch in size (5 cc in volume), which contains 40% copper (as metal) and 26.5% chromium (as metal), underwent reduction treatment in the same manner as in Example 1. The same reaction as in Example 1 was carried out except that the molar ratio of hydrogen to 1,4-butanediol was 3, the reaction pressure was 0 $kg/cm^2G$, and the W.H.S.V. of 1,4-butanediol was 3.9 $h^{-1}$.

It was found that the conversion of 1,4-butanediol was 71.2 mol%, the selectivity of gamma-butyrolactone was 97.3 mol%, and the yield of gamma-butyrolactone was 69.3 mol%.

Comparative Example 2

The same procedure as in Comparative Example 1 was repeated for catalyst reduction and reaction, except that the catalyst was replaced by a commercial copper-zinc oxide catalyst ("N-211" made by Nikki Kagaku Co., Ltd.) (5 cc in volume), which contains 50% CuO and 45% ZnO.

It was found that the conversion of 1,4-butanediol was 71.5 mol%, the selectivity of gamma-butyrolactone was 93.1 mol%, and the yield of gamma-butyrolactone was 66.6 mol%.

Comparative Example 3

The same procedure as in Comparative Example 1 was repeated for catalyst reduction and reaction, except that the catalyst was replaced by a commercial copper-chromiummanganese oxide catalyst ("G-89" made by Nissan Gardlar Shokubai Co., Ltd.) (5 cc in volume), which contains 38.9% copper, 37. 3% chromium, and 3. 6% manganese (all as metal) It was found that the conversion of 1,4-butanediol was 86.9 mol%, the selectivity of gamma-butyrolactone was 97.9 mol%, and the yield of gamma-butyrolactone was 85.1 mol%.

This comparative example should be compared with Example 6 of the invention since the conditions of dehydrogenation are substantially similar except for the different catalyst.

Table 1

| | Example 2 | Example 3 | Example 4 | Example 5 | Example 6 |
|---|---|---|---|---|---|
| **Reaction Conditions** | | | | | |
| Reaction temperature (°C) | 200 | 200 | 200 | 200 | 230 |
| Reaction pressure ($kg/cm^2G$) | 1 | 3 | 0 | 3 | 0 |
| $H_2$/1,4-butanediol (molar ratio) | 6 | 6 | 2 | 3 | 3 |
| W.H.S.V. of 1,4-butanediol ($h^{-1}$) | 3.6 | 3.6 | 3.6 | 3.6 | 3.6 |
| **Results** | | | | | |
| Conversion of 1,4-butanediol (mol%) | 80.1 | 82.5 | 90.3 | 99.6 | 96.3 |
| Selectivity of gamma-butyrolactone (mol%) | 98.4 | 99.1 | 98.7 | 98.9 | 97.7 |
| Yield of gamma-butyrolactone (mol%) | 78.8 | 81.8 | 89.1 | 98.5 | 94.1 |

## Claims

1. A process for producing gamma-butyrolactone by catalytic dehydrogenation of 1,4-butanediol in the gas phase in the presence of a catalyst containing copper, chromium, manganese and barium and in the presence of hydrogen.

2. The process of claim 1 wherein the catalyst is the reduction product of a mixture of copper oxide, chromium oxide, manganese oxide and barium oxide.

3. The process of claim 1 wherein the dehydrogenation is carried out at a temperature of 190-240 °C, a

reaction pressure of 0.5-4 $kg/cm^2G$ and an $H_2$/1,4-butanediol molar ratio of about 2-6.

European Patent Office

# EUROPEAN SEARCH REPORT

Application Number

EP 91 30 3039

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| A | EP-A-0 184 055 (HÜLS AKTIENGESELLSCHAFT)<br>* the whole document; in particular abstract, claims 1-4 *<br>--- | 1 | C07D307/33 |
| A | CHEMICAL ABSTRACTS, vol. 108, no. 16, 18 April 1988, Columbus, Ohio, US;<br>abstract no. 133838Z,<br>A. GHITA-DUMINICA ET AL.: 'Catalytic manufacture of gamma-butyrolactone'<br>page 109 ;column 2 ;<br>* abstract *<br>& RO-A-91 930<br>--- | 1 | |
| A | CHEMICAL ABSTRACTS, vol. 94, no. 7, 16 February 1981, Columbus, Ohio, US;<br>abstract no. 46506P,<br>T. F. OVCHINNIKOVA ET AL.: 'Preparation of gamma-butyrolactone from 2-butene-1,4-diol'<br>page 507 ;column 1 ;<br>* abstract *<br>& NEFTEKHIMIYA<br>vol. 20, no. 6, 1980, USSR<br>pages 858 - 863;<br>--- | 1 | TECHNICAL FIELDS SEARCHED (Int. Cl.5 ) |
| D,A | WORLD PATENTS INDEX LATEST<br>Week 8650,<br>Derwent Publications Ltd., London, GB;<br>AN 86-329466<br>& JP-A-61 246 173 (IDEMITSU PETROCHEM CO. LTD.)<br>1 November 1986<br>* abstract *<br>----- | 1-3 | C07D |

The present search report has been drawn up for all claims

| Place of search | Date of completion of the search | Examiner |
|---|---|---|
| THE HAGUE | 28 NOVEMBER 1991 | PAISDOR, B. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document